# EUROPEAN PATENT APPLICATION

(11) **EP 1 953 219 A1**
(43) Date of publication of application: **06.08.2008**
(21) Application number: 07101406.2
(22) Date of filing: 30.01.2007
(51) Int. Cl.: C12M 1/16, C12M 1/22

(54) **Method for providing storable plates filled with a biological culture medium**

(71) Applicant: Becton, Dickinson & Company, Franklin Lakes, NJ 07417-1880 (US)
(72) Inventor: Gomez, Anselmo Quijada, 69517, Gorxheimertal (DE); Hammann, Rainer, 69257, Wiesenbach (DE); Wischnewski, Frank, 69231, Rauenberg / OT Malschenberg (DE)
(74) Representative: von Kirschbaum, Alexander

(57) **Abstract**

According to the invention a method for providing storable plates containing a biological culture medium includes the steps:
providing a biological culture medium to at least one plate (10),
decreasing solidification time for the biological culture medium by use of a conditioned air stream
removing excess water from the surface of the biological culture medium by use of an warm, dry air stream, and
sealing the at least one plate (10) by use of a plastic film (12).

## Description

The invention relates to a method for providing storable plates filled with a biological culture medium.

### Background

Plates containing biological culture media, e.g. MacConkey Agar, Columbia Agar and many other media formulations, are often used in biological research and in clinical laboratories.. Petri plates are a common form of such plates. Agar is often used as the gelling agent for the biological culture medium. A stack of Petri dishes is often wrapped by a cellophane film. A number of manufacturers sell plates that are pre-filled with biological culture media (prepared plated media (PPM)).

The storage time of most prepared plate media is limited due to shrinkage of the medium. Shrinkage of the medium may be caused by the loss of water contained in the agar gel by evaporation of water through the cellophane film during storage. Shrinkage may influence the appearance of the medium only, but may also influence the microbiological performance due to an increase of the concentration of selective or other ingredients when water escapes from the medium. In the packed stacks of e.g. ten plates, water loss is strongest in the uppermost and lowermost plates, while it is weakest in the middle of the stack. Shrinkage also is a function of the storage temperatures and the air humidity. The medium will shrink faster at higher temperatures and at lower air humidity than at lower temperature and higher air humidity. Since the evaporation is higher at ambient temperature than in refrigerated conditions, cellophane packed plates usually need to be stored refrigerated. The storage time or storage time of refrigerated dishes packed with cellophane film is limited to eight to fourteen weeks on average. For purposes of this patent, storage time and storage time are used interchangeably.

Another disadvantage of cellphone film is that the moisture of the plates and the stack varies. Furthermore, during the transportation of a packed stack of plates, condensation water may flow out of the dishes so that the outer surface of the dishes is wet. Users of prepared plated media having condensation water must often dry to places before they can be used. An improved method for providing storable plates containing biological culture medium is needed. A method to reduce the amount of condensation water in storable plates is also needed.

### Brief Summary of the Invention

It is an object of the invention to provide a method for providing storable plates containing biological culture media so that the storage time is increased. It is a further object of the invention to provide a method for reducing the amount of condensation water in storable plates.

This and other objects are achieved by a method according to claim 1.

According to the invention, a method for providing storable plates containing a biological culture medium is proposed. This method includes the following steps:
- providing a biological culture medium to at least one plate,
- decreasing solidification time for the biological culture medium by use of a conditioned air stream
- removing excess water from the surface of the biological culture medium by use of an warm, dry air stream, and
- sealing the at least one plate by use of a plastic film.

### Detailed Description

In one embodiment of the invention, a first step of the method for providing storable plates is to fill or partially fill at least one plate, particularly a Petri dish, with a biological culture medium. The used media is e.g. MacConkey Agar, Columbia Agar and many other media formulations. Thereafter, the medium is allowed to solidify excess condensation water is removed from the surface of the medium. According to the invention this is done by use of an air stream. After the water is removed by the air stream so that the moisture of the media is preferably in a specific range, the at least one plate is closed with the lid of the dish and is then sealed by use of a plastic film. Since according to the invention the excess water is removed from the surface of the media, it is possible to use a plastic film to pack the plates, whereby the plates can be sealed by the plastic film. Thus, the medium does not dry during storage. Dependent on the film that is used to pack the plates, the loss of moisture during the storage of the plates can be minimized to a water vapour permeability of 2,6 g/m²/24h at Klima D (23 +/-1)°C (85+/-2)% rel. humidity (see DIN 53122).

Preferably a multi-layered or mono-layered film (foil) is used. The film may have a barrier layer.

Since the moisture of the media is in a specific predetermined range after it has been dried by the air stream, the moisture of the media does not vary from plate to plate. Since the plate, particularly a stack of dishes, is sealed by packing the plates by use of a plastic film, the packed plates can be stored at room temperature, if the stability of the ingredients of the medium allows. Another advantage of the invention is that the packed plates can be stored up to fourteen weeks, particularly up to thirty weeks. Furthermore, the plates can be packed immediately after the production, because there is no need to wait for the passive evaporation of condensing water that occurs during the storage of the media.

After the drying step, in which the surface water has been removed from the media by an air stream, the amount of condensation water on the medium surface per plate is preferably in the range of 0 to 0.15 g.

The air stream used to remove the surface water from the media has preferably a relative humidity of 0 - 20 %, particularly 5 - 10 %. The temperature of the air stream is preferably within the range of 40 - 60 °C and particularly 50 - 55 °C. Furthermore, the velocity of the air is within the range of 2 - 4 m/sec and particularly 2.5 - 3 m/sec. To obtain the desired moisture of the media within the plate, the air stream is directed onto the media, for preferably 1 - 3 min., particularly 1.5 - 2 min. Furthermore, the preferred direction of the air stream is parallel and in line with the movement of the conveyor.

In a preferred embodiment of the method according to the invention, the step of filling the plates is an automated continuous process. Preferably, the drying step and/ or the packaging step are also automated. Thus, it is preferred that the plates are filled while they are transported on a conveyor belt. Furthermore, the removing of the surface water is performed preferably while the plates are arranged on a conveyor belt. This can be done during the adjustable movement of the conveyor belt. Alternatively, the movement of the plates can be interrupted to remove the surface water by the air stream.

Preferably, the plates are cooled after filling the plates with the biological culture media. This is provided to minimize the time required to solidify the media. Preferably, the plates are cooled by a conditioned air stream.

After the surface water has been removed according to the invention by use of a specific air stream, the plate, preferably a stack of plates, e.g. a stack of Petri dishes, is closed with its lid and is packed. This is performed by use of two pre-shaped plastic film elements. Each of these pre-shaped plastic film elements covers preferably one half of the plate and one half of the stack of dishes respectively. Each plastic film element has preferably a plane edge surrounding the pre-shaped element. The plane edges of the two pre-shaped plastic film elements are located opposed to each other and contact each other so that the package can be closed by connecting the two edges. This can be effected by use of an adhesive or by thermo sealing.

Before removing the excess water by use of an air stream as described above, it is possible to decrease the solidification time for the gel by use of a conditioned air stream. Thus, it is possible to use a warm and dry air stream not only to remove excess water, but also to solidify the gel.

### Brief description of the drawings

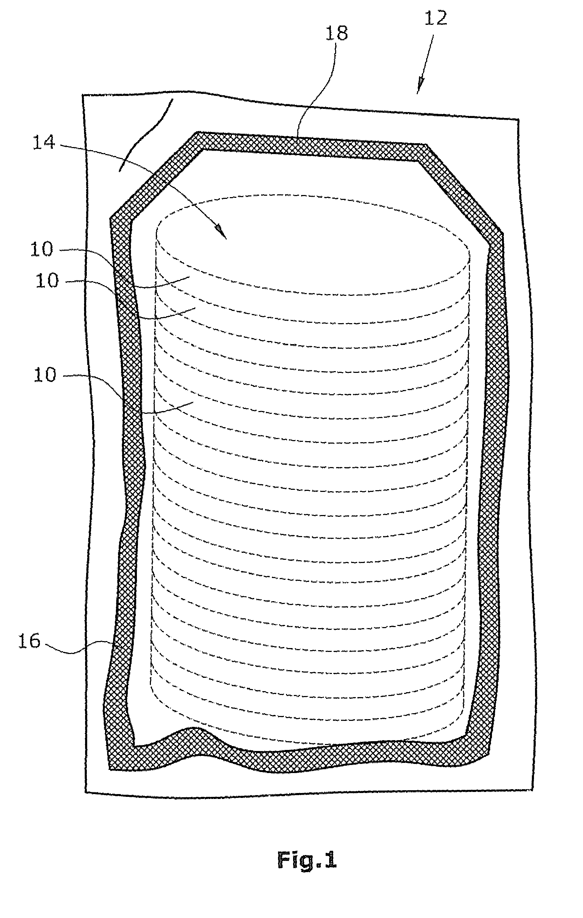

The invention will be described in more detail in view of the enclosed drawings and diagrams. Within the drawings
Fig. 1 shows a preferred embodiment of packed Petri dishes,
Fig. 2-8 show diagrams of test 1,
Fig. 9, 10 show diagrams of test 2, and
Fig. 11 - 17 show diagrams of test 3.

After the surface water is removed from the agar gel, ten Petri dishes 10 are placed one over the other, forming a stack of dishes. The stack of dishes 10 is arranged within two pre-shaped plastic film elements 12. The two elements 12 are identical, whereby each element has a half-cylindrically shaped part 14 surrounding one half of the stack. This half-cylindrically shaped part 14 is surrounded by an edge 16. Thus, the edges 16 are completely surrounding the half-cylindrically shaped part. The two edges 16 of the two pre-shaped plastic film elements 12 are located opposed to each other. The two edges 16 are connected to each other by a seal 18. The seal 18 is made by thermo sealing, i.e. by supplying heat.

The new method was tested by performing the following tests:

### Example 1:

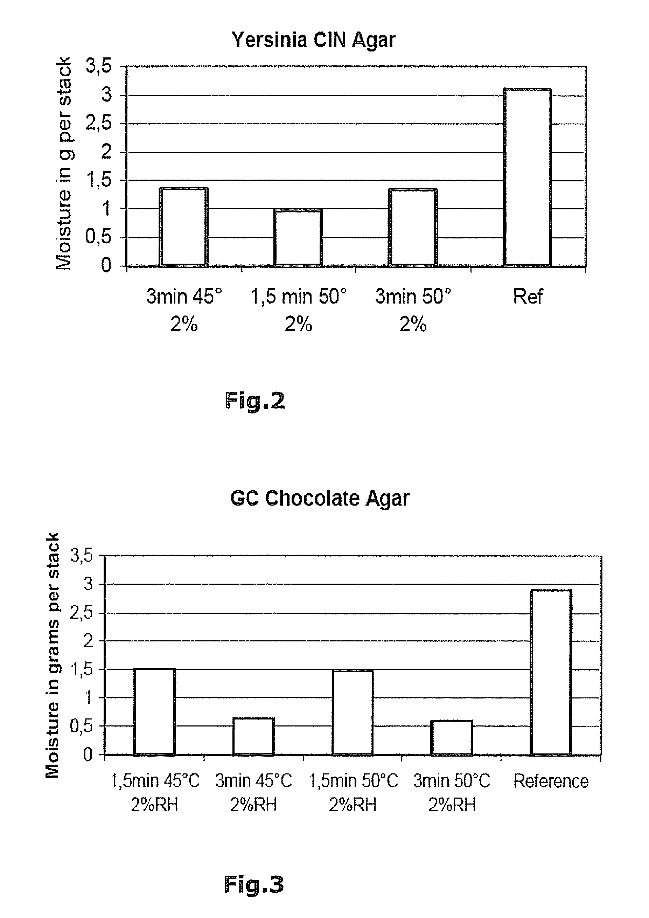

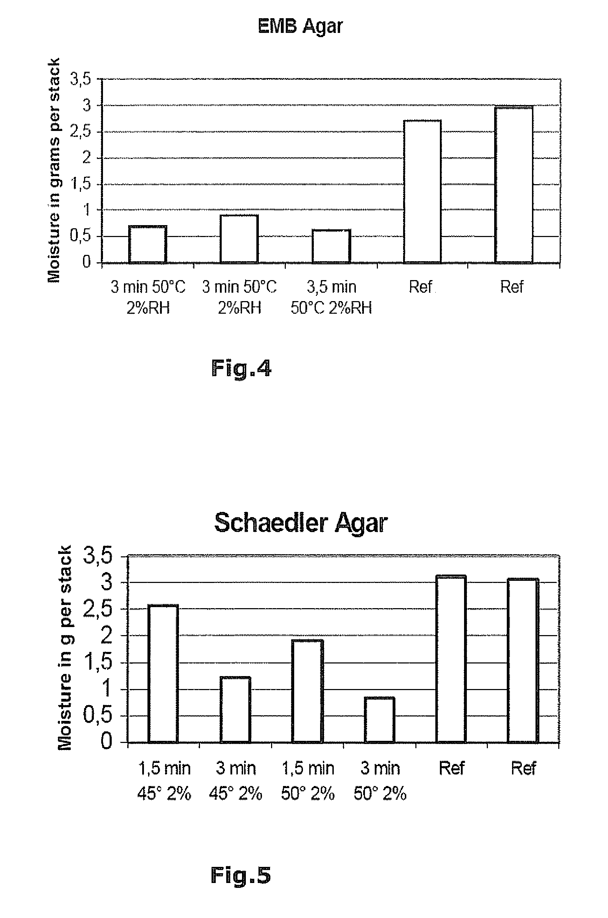

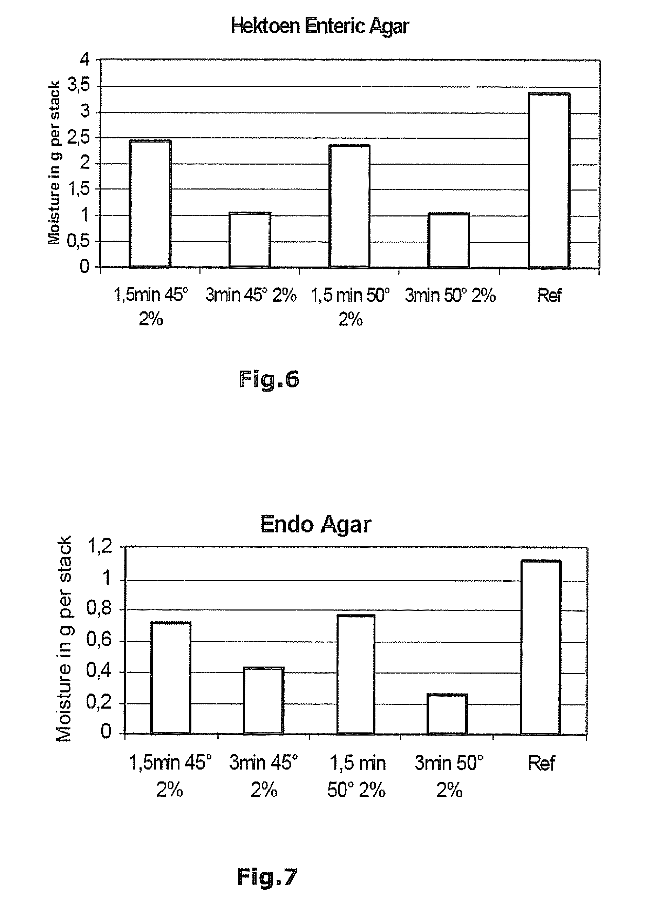

In order to avoid stress to media, the drying temperatures should not be higher than the pouring temperature of the media (e.g. 46 to 52 °C), air temperatures in this range were tested. The air humidity, on the other hand, is not limited; therefore, very dry air (0 - 5 % relative humidity [=RH]) was used. It must be mentioned that,in the experiments, both temperature and air humidity varied slightly from the setpoint. However, as can be seen from Fig. 2-8, the drying process was effective for all PPM tested, as compared to the reference stacks which were shrink-wrapped without drying. Drying efficiency was dependent on both the drying time and the temperature. It also became evident that lids should be removed during the drying process to obtain an efficient drying within an acceptable time period (=several minutes). The acceptable time period depends on the efficiency and performance of the dryer and the machine speed. A range of 1.5 to 3 min is acceptable for this process.

Also, it became evident that very moist media (e.g. GC Chocolate Agar, Hektoen Enteric Agar, Yersinia Agar) need slightly longer drying times and/ or higher temperatures for drying than relatively dry media (e.g. Columbia Agar with 5% SB, Endo Agar). The "very moist" media need the maximum drying time (3 min). The threshold of acceptable excessive moisture was 1.5 g water per stack of 10 plates.

More than 20 different media were tested during the first series (see Table 1), and all of them were successfully dried with hot dry air. In the later tests, in which dried plates were also subjected to microbiological performance and storage time tests, additional media were tested.

### Table 1:

Media included in the first test series with hot dry air in the climate-controlled incubator.

| Medium Name | Conditions effective in reducing moisture to or below 1.5 ml per stack at... | | Conditions effective in reducing moisture to 1.0 ml or below |
|---|---|---|---|
| | 45° | 50° | 45 or 50° C |
| Bromcresol Purple Lactose | yes | yes | yes |
| CampyBap | nt* | yes | |
| Campy Bloodfree | yes | yes | yes |
| CHROMagar Orientation | yes | yes | yes |
| CHROMagar Salmonella | yes | yes | yes |
| CLED | yes | yes | yes |
| Columbia 5% SB | yes | yes | yes |
| Columbia III 5% SB | yes | yes | yes |
| EMB | yes | yes | yes |
| Endo | yes | yes | yes |
| GC Chocolate | yes | yes | yes |
| Hektoen Enteric | yes | yes | yes |
| MacConkey II | yes | yes | no |
| Mannitol Salt | yes | yes | yes |
| MH Chocolate | yes | yes | no |
| Mueller Hinton II | yes | yes | yes |
| Mycoplate MS | yes | yes | yes |
| Sabouraud Glucose | yes | yes | yes |
| Salmonella Shigella | yes | yes | no |
| Schaedler | yes | yes | yes |
| TSA II 5% SB | yes | yes | yes |
| XLD | yes | yes | no |
| Yersinia | (yes) | yes | (no)* |

| | | | |
|---|---|---|---|
| Nt= not tested | | | |

From these tests, it became evident that all media can be dried with this technique. Slight modifications in the drying temperature, time, or air stream velocity (this parameter can be modified) are necessary to dry wet media and to avoid desiccation of rather dry media.

### Test 2

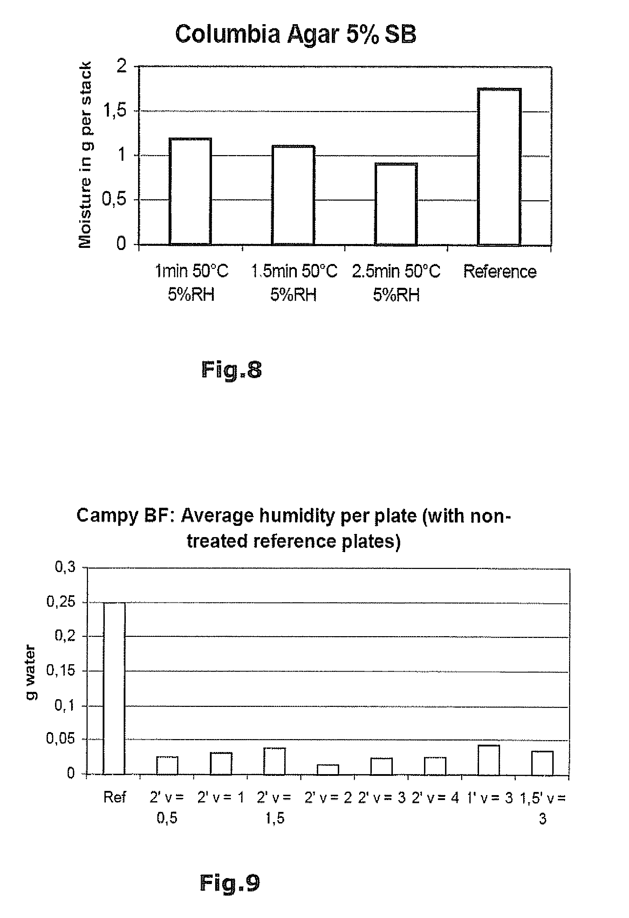

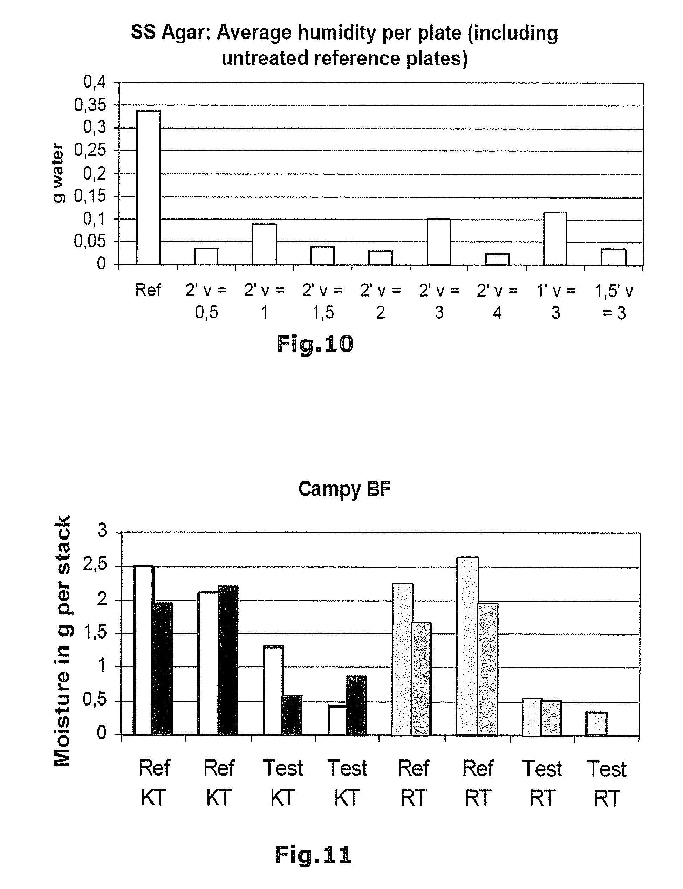

A convection dryer was used for additional drying tests. Freshly prepared media (prepared manually) were directly after solidification placed in the dryer which has a moving conveyor (speed adjustable) and allows adjustment of the drying temperature and air humidity. The results are shown in Figures 9 and 10. As can be seen, drying at 50 °C and 5 % RH and with variable air speed (v, [m/sec]) is effective for removing the excessive moisture. It must be noted that these plates were poured manually, which results in different fill weights and in different amounts of excessive water - therefore the effect of drying is not always in direct relation to the drying time or air speed. However, drying was effective for all three media tested (Campy BF, Salmonella Shigella and Sabouraud Glucose Agar).

### Test 3

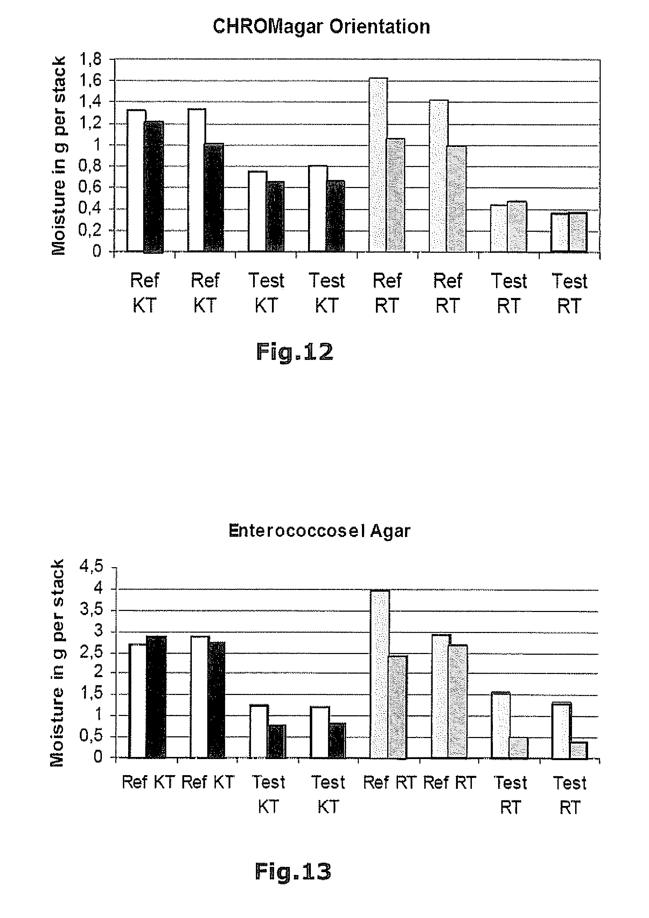

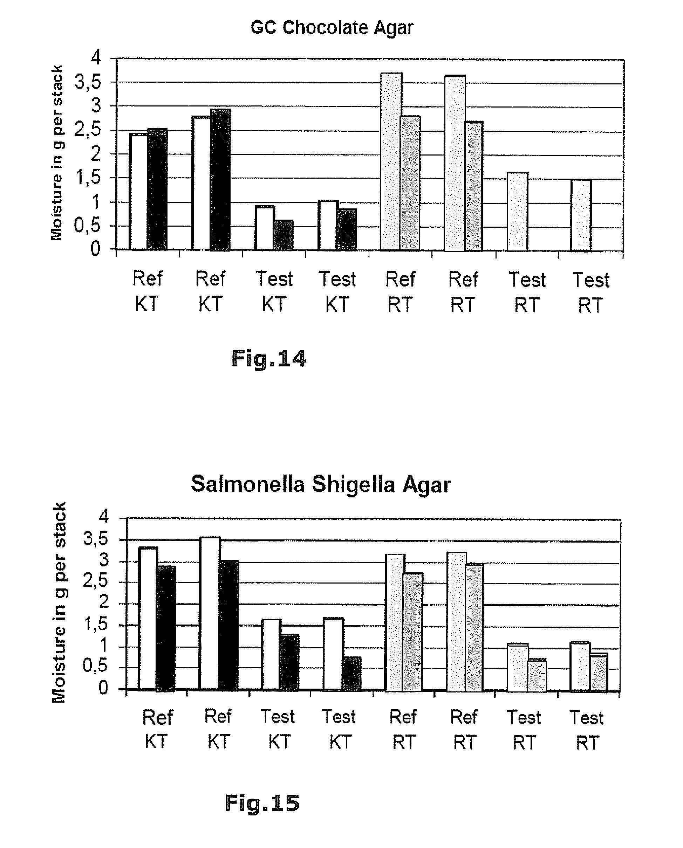

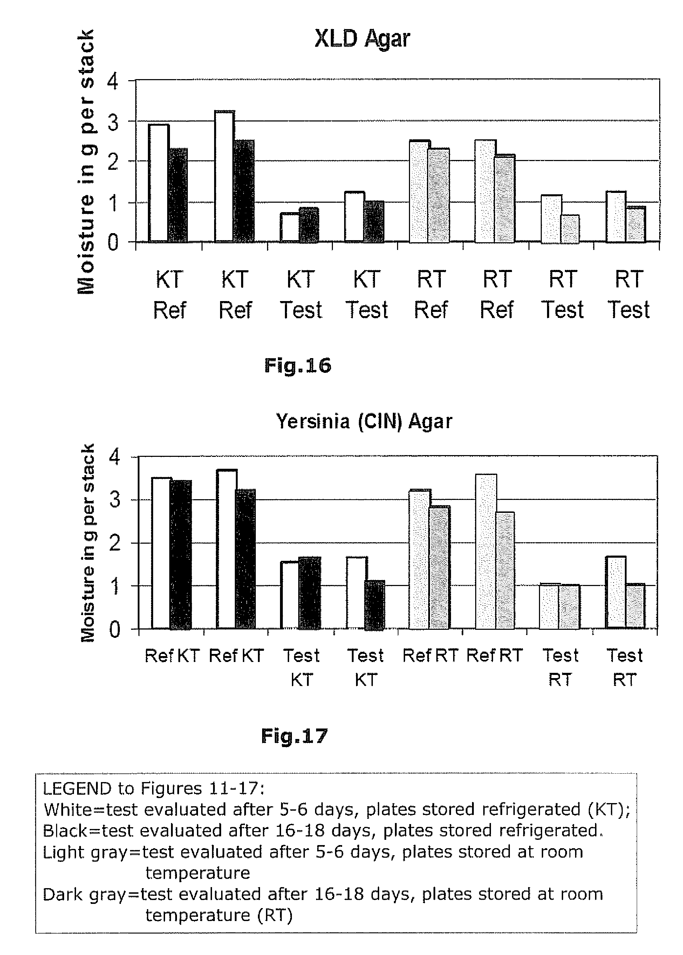

Next, an industrial convection test dryer was installed in the Manufacturing Packaging area. Plates were manually transferred directly from the filling machine into the drying tunnel. The drying conditions were 51 - 52 °C, 2 - 5 % RH, air speed: 1.5 m/sec, dwelling time: between 1 and 2 min. After drying, plates were shrink-wrapped in bags, and were kept for four hours at room temperature for 5 - 6 days and for 16 - 18 days. As can be seen from Fig. 11 - 17, plates were well dried with this drying tunnel and all dried plates were around or below the 1.5 g threshold while the undried reference plates were significantly above it. Slight adjustments may be necessary for the final drying conditions when very wet media are produced, but the drying procedure apparently works for all of them.

## Claims

1. A method for providing storable plates containing a biological culture medium including:
providing a biological culture medium to at least one plate (10),
decreasing solidification time for the biological culture medium by use of a conditioned air stream
removing excess water from the surface of the biological culture medium by use of an warm, dry air stream, and
sealing the at least one plate (10) by use of a plastic film (12).

2. The method according to claim 1, wherein the medium is dried by the air stream to a moisture range of 0 - 10 % RH and/or 0 - 0,15 g water per plate.

3. Method according to claim 1 or 2, wherein the air stream has a temperature of 40 - 60 °C, preferably 40 - 55 °C.

4. The method according to one of the claims 1-3, wherein the air stream has a relative humidity of 5 - 20 %, preferably 5 - 10 %.

5. The method according to one of the claims 1-4, wherein the air stream has a velocity of 2 - 4 m/sec, preferably 2.5 - 3 m/sec.

6. The method according to one of the claims 1 - 5, wherein the drying time to remove excess water is 1 - 3 min, preferably 1,5 - 2 min.

7. The method according to one of the claims 1-6, wherein the plates (10) are filled in a continuous process while the plates are transported on a conveyor belt.

8. The method according to one of the claims 1 - 7, wherein the plates (10) are cooled after filling, particularly by cooling the conveyor belt, preferably with a conditioned air stream.

9. The method according to one of the claims 1 - 8, wherein the plates (10) have a tray- or dish-like shape.

10. The method according to one of the claims 1 - 9, wherein the medium is solidified before removing the excess water.

11. The method according to one of the claims 1 - 10, wherein a stack of plates (10) is sealed commonly.

12. The method according to one of the claims 1 - 11, wherein the packing is performed by use of two pre-shaped plastic film elements (12) being closed by a thermo seal (18).
